# EUROPEAN PATENT SPECIFICATION

(11) **EP 2 637 582 B1**
(45) Date of publication and mention of the grant of the patent: **23.08.2017**
(21) Application number: 11779707.6
(22) Date of filing: 11.11.2011
(51) Int. Cl.: A61B 17/70

(54) **SPINAL FIXATION SYSTEM**
FIXATIONSSYSTEM FÜR DIE WIRBELSÄULE
SYSTÈME DE FIXATION RACHIDIENNE

(30) Priority: 22.11.2010 US 458334 P; 12.11.2010 US 412825 P
(43) Date of publication of application: 18.09.2013
(73) Proprietor: Aesculap AG, 78532 Tuttlingen (DE)
(72) Inventor: GARCIA, Rolando, Golden Beach Florida 33160 (US); HAAS, Alexander, 78166 Donaueschingen (DE); BEGER, Jens, 78532 Tuttlingen (DE); KLINGSEIS, Susanne, 88400 Biberach (DE); SCHULTZ, Robert, 78532 Tuttlingen (DE); STOERK, Claudia, 78576 Emmingen (DE); DAIBER, Wolfgang, 72336 Balingen (DE); HOEGERLE, Roland, 78532 Tuttlingen (DE); PRAEDEL, Christian, 78532 Tuttlingen (DE); WIMBER, Johannes, 78333 Stockach, (DE)
(74) Representative: Hoeger, Stellrecht & Partner Patentanwälte mbB
(86) International application number: PCT/EP2011/069895
(87) International publication number: WO 2012/062889

(56) References cited:
- WO-A1-2006/102428
- WO-A1-2009/127041
- WO-A1-2010/114925
- DE-A1- 4 411 974
- DE-A1- 10 326 690
- DE-B3-102006 021 025
- US-A- 5 496 318
- US-A1- 2003 040 746
- US-A1- 2006 247 640
- US-A1- 2008 086 212
- US-A1- 2008 108 990
- US-A1- 2009 265 006

## Description

The invention relates to a surgical fixation system.

Furthermore, the disclosure relates to a spacer element for a surgical fixation system.

The disclosure also relates to a use of an implant.

Moreover, the disclosure relates to a method for stabilising spinous processes.

US 5,496,318 describes a spinal fixation device and method for the stabilisation of the spine after surgical procedures such as those related to degenerative disc diseases. The device comprises a spacer, which is placed between adjacent vertebrae when installed, and a locking mechanism attached to the spacer. The locking mechanism attaches the device to the spinous processes of adjacent vertebrae of the spine in a manner which is non-invasive with respect to the vertebrae to which it is attached.

US 2006/0247640 A1 discloses devices and methods for supporting adjacent spinous processes including opposing plates movable toward one another along a cross post to contact opposite sides of each of the spinous processes, and including a spacer member about the cross post contacting the adjacent surfaces of the spinous processes to resist movement of the spinous processes toward one another under spinal extension motion.

Further surgical fixation systems including a spacer member are disclosed in US 2008/0086212 A1, US 2003/0040746 A1 and US 2008/0108990 A1.

WO 2010/114925 A1, WO 2009/127041 A1 and US 2009/0265006 A1 also describe surgical fixation systems for stabilising spinous processes.

A surgical fixation system in accordance with the invention comprises the features of claim 1.

A fixation system of this type solves the object to provide a fixation system for spinous processes having a structurally simple configuration. The at least one spacer element can be used to support the spinous processes relative to one another, and it can be fixed in a structurally simple manner by the fixing elements in the intervertebral space. In particular, it can further be provided that the fixing elements are engaged only by clamping with the spinous processes and are not screwed to them. The danger of a screw being pulled out, such as can occur in the fixation systems known from the prior art, can be thereby avoided. The spacer element, for example, allows for the fixing elements to take up a well-defined position relative to one another, and for the support of the spinous processes. The fixation system according to the invention also allows, in a structurally simple manner, a modular configuration of instruments for the fixation of the spinal column according to the invention, wherein it is possible for the at least one spacer element and the fixing elements to be available separately from one another, in each case, for example in different sizes. An individual adaptation to the size of the spinous processes to be stabilised with one another in each case can therefore be carried out in a simple manner by a suitable combination of one or more spacer elements with fixing elements.

"Vertebral body" in the present case designates both the vertebra as a whole and also the vertebral body in the narrower sense ("corpus vertebrae").

"Adjacent vertebral bodies" primarily indeed means "directly adjacent vertebral bodies", but, in the present case, is not restricted to directly adjacent vertebral bodies.

Anatomical position and direction details such as, for example "medial", "lateral", "cranial", "caudal", "dorsal", "ventral", "sagittal plane", "anterior", posterior" or the like, which relate to features of the fixation system, are to be understood, in the present case, as applied in the implantation position of the fixing elements, in which implantation position the fixation system is fixed to the spinous processes as intended.

The at least one spacer element comprises or forms a cranial contact face for a spinous process, for example a planar, a saddle-shaped or a groove-shaped contact face.

The at least one spacer element also comprises or forms at least one caudal contact face for a spinous process, for example a planar, a saddle-shaped or a groove-shaped contact face. In combination with the last-mentioned implementation, the spinous processes can be supported against one another in the cranial-caudal direction.

It is provided that the at least one spacer element comprises or forms at least one anterior contact face for at least one vertebral body, in particular dorsally, for at least one lamina arcus vertebrae, and optionally for the processus articularis superior and/or the processus articularis inferior at least of one of the vertebral bodies. A facet joint formed between adjacent vertebral bodies, for example, can be stabilised thereby. The contact face can be in the form of a strip.

It may, in particular, be provided that at least one spacer element can be fixed in positively-locking and/or force-locking manner.

For example, the at least one spacer element can be fixed by clamping, so the fixation system can be easily handled.

Furthermore, it is possible for the at least one spacer element to engage, in particular by latching, at least in the implantation position, with at least one fixing element. For this purpose it and/or the at least one fixing element can have engagement members.

The at least one spacer element may comprise or form at least one lateral contact face for at least one fixing element.

Anterior and/or posterior to the at least one spacer element, a receiving space can be formed between the fixing elements, into which receiving space, for example, bone or a bone replacement material can be inserted for the supplementary stabilisation of the spinous processes relative to one another.

It is possible for at least one spacer element to form a receiving space for bone and/or bone replacement material.

Furthermore, it may be provided that at least one spacer element engages in the implantation position of the fixation system with at least one spinous process. For example, the spacer element comprises engagement members in the form of cutting edges, for example a thread. For this purpose, the spacer element can form a screw.

It may be provided that the at least one spacer element is rigid.

Alternatively, it may be provided that the at least one spacer element is deformable.

In particular, the at least one spacer element can be resiliently deformable, for example in the cranial-caudal, in the dorso-ventral and/or in the medial-lateral direction.

The at least one spacer element preferably acts upon the fixing elements in the implantation position with forces directed away from one another and is thereby reliably fixed between the fixing elements.

It may be provided that the size of at least one spacer element is variable, specifically in the cranial-caudal, in the medial-lateral and/or in the dorso-ventral direction.

In particular, an adjusting device for varying the size of the at least one spacer element may be provided.

The at least one spacer element is advantageously formed in one piece.

For example, the at least one spacer element is at least partially plate-like, it being possible for it to be plate-like overall, in particular.

With the plate-like portion, the at least one spacer element is oriented, for example, in a sagittal plane or parallel to a sagittal plane, with the fixation system being fixed to the spinous processes as intended.

The at least one spacer element may be lattice-shaped and thus have osteointegrative properties.

Furthermore, the at least one spacer element or a group of spacer elements, a "spacer package", may be bellows-shaped.

The at least one spacer element may also be cushion-like and be formed as a gel-cushion or the like.

It may also be provided that the at least one spacer element is cuboidal and forms a hollow body, which, for example, can be filled with resilient elements.

It is possible for the at least one spacer element to comprise at least one engagement element for an engagement of the at least one spacer element with a further spacer element, so that the two spacer elements can adopt a defined position relative to one another. The at least one engagement element is, for example, a projection, in particular a strip or a rib.

A structurally simple fixation system comprises precisely one spacer element.

Another type of fixation system may comprise two or more spacer elements. These may together form an ensemble of spacer elements, a so-called "spacer package".

The two or more spacer elements may be formed identically or at least two spacer elements may be formed identically.

In a corresponding manner, the two or more spacer elements may be formed differently, or at least two spacer elements may be formed differently.

It is possible for two or more spacer elements to abut against one another, wherein they can, in particular, rest flat on one another, for example in a sagittal plane, with the fixation system being fixed to the spinous processes as intended.

Furthermore, it is possible for two or more spacer elements to engage with one another, in particular in positively-locking manner.

For example, it is possible for two or more spacer elements to abut against one another or engage with one another with the medial-lateral direction, but it may also be provided that two or more spacer elements engage with one another or abut against one another in the cranial-caudal direction or in the dorso-ventral direction.

The at least one spacer element may be at least partially resorbable.

The at least one spacer element may be made at least partially from an osteointegrative material or be osteointegratively coated.

For example, the at least one spacer element is made from metal, for example from titanium.

The at least one spacer element may also be made from a plastics material, for example from PEEK.

It is also possible to make the at least one spacer element from bone material or a bone replacement material, such as, for example, hydroxyapatite.

The at least one spacer element may be at least partially coated, for example by vacuum-coating with high-purity titanium powder, which forms a rough microporous titanium layer for improved anchoring to the bone on the surface of the spacer element (so-called "Plasmapore" coating below). "Plasmapore" is a registered trademark of Aesculap AG and Aesculap Inc.

It is possible for at least one spacer element to be engaged or engageable with the securing device, in particular when the fixing elements adopt the implantation position.

In particular, the at least one spacer element can comprise or form at least one opening through which the securing device engages.

It is furthermore possible for the at least one spacer element to comprise or form at least one recess, in which the securing device engages.

Each fixing element in the implantation position is preferably engageable with two spinous processes in order to achieve a reliable stabilisation by the fixation system.

At least one fixing element can be planar.

Further, at least one fixing element can be adapted to be oriented in a sagittal plane or substantially in a sagittal plane when the fixation system is fixed to the spinous processes as intended.

It may be provided that at least one fixing element is rigid.

Furthermore, at least one fixing element may be deformable, in particular resiliently deformable. It may, for example, comprise defined deforming regions, for example bending zones, to allow an adaptation to the spinous processes.

It is possible for at least one fixing element to be rectangular.

It can also be provided that at least one fixing element is step-like with two portions offset relative to one another in the cranial-caudal direction. This is advantageous if a plurality of spinous processes are to be stabilised relative to one another. The offset may, for example, be approximately one third or approximately half of the cranial-caudal extent of the fixing element.

For engagement in the spinous processes, at least one fixing element has, for example, projections in the lateral-medial direction.

No projections are advantageously formed in the region of the intervertebral space, for example in order to avoid damage to the at least one spacer element or injury to the patient. For this purpose, the fixing element can comprise sections having projections at opposite ends and a section without projections therebetween.

The projections are, for example, arranged at one edge of the fixing element.

It may furthermore be provided that the projections enclose an acute angle with a plane defined by the fixing element.

It is possible for the projections to be deformable in a defined manner. For example, they can be transferred into an engagement position, in that the angle, which they enclose with the fixing element, is increased.

At least one fixing element may be made from metal, for example from titanium. A resorbable fixing element, a fixing element made of a plastics material and an at least partially coated fixing element are also conceivable.

The securing device is preferably a latching device, having one or more latching elements.

At least one latching element can be, for example, be a clamp rod or tie rod.

The latching device can, in particular, be a self-locking latching device, which facilitates implantation of the fixation system.

The latching device may, for example, have at least one latching element, which is fixed to a fixing element and on which the other fixing element can be latched, so that it can be easily operated.

In addition or alternatively, separate locking members can be provided for latching to the latching elements, so that a fixing element can be fixed thereto.

The at least one latching element can engage through the at least one spacer element or engage therein, so that the at least one spacer element can be fixed on the securing device.

Furthermore, the at least one latching element can engage through at least one fixing element or engage therewith.

The latching device can comprise two or more latching elements, which can be formed identically or differently.

Moreover, as mentioned at the outset, the disclosure relates to a spacer element. A spacer element according to the disclosure can comprise one or more features which the at least one spacer element of the fixation system according to the invention or one of the preferred embodiments of the fixation system has and is adapted to be used in a fixation system according to the invention.

As mentioned at the outset, the disclosure also relates to a use. According to the disclosure, a sternal closure device described in the document DE 103 26 690 B4 or a fixing device described in the document DE 10 2006 021 025 B3 can be used as a fixation system for stabilising spinous processes of adjacent vertebral bodies relative to one another. The sternal closure device or the fixing device does not need to agree in all its details with the sternal closure device known from DE 103 26 690 B4 or with the fixing device known from DE 10 2006 021 025 B3. For example, a difference is possible with regard to the size, ratio of the longitudinal side to the transverse side of the fixing elements, arrangement of the fixing projections on the fixing elements, arrangement of the securing pins relative to the fixing elements or the like. At least one spacer element can be positioned between the fixing elements in the intervertebral space.

When using the sternal closure device or the fixing device, a fixing element is advantageously transferred in each case into an implantation position and engaged laterally with at least one spinous process, the fixing elements being arranged on different lateral sides of the spinous processes.

The disclosure also relates to a method, in which a sternal closure device described in the document DE 103 26 690 B4 or a fixing device described in DE 10 2006 021 025 B3 is used to stabilise spinous processes of adjacent vertebral bodies relative to one another, with at least one spacer element being positioned between two fixing elements of the fixing system in the intervertebral space.

The following description of preferred embodiments of the disclosure is used for a more detailed description in conjunction with the drawings, in which:
Fig. 1: shows a perspective view of a first preferred embodiment of a fixation system according to the disclosure;
Fig. 2: shows a perspective view of a second preferred embodiment of a fixation system according to the disclosure;
Fig. 3: shows a perspective view of a third preferred embodiment of a fixation system according to the disclosure;
Fig. 4: shows a perspective view of a fourth preferred embodiment of a fixation system according to the disclosure;
Fig. 5: shows a perspective view of a fifth preferred embodiment of a fixation system according to the disclosure;
Fig. 6: shows the fixation system from Fig. 1 when being fixed to two spinous processes;
Fig. 7: shows the fixation system from Fig. 2, fixed to two spinous processes;
Fig. 8: shows the fixation system from Fig. 3 when being fixed to two spinous processes;

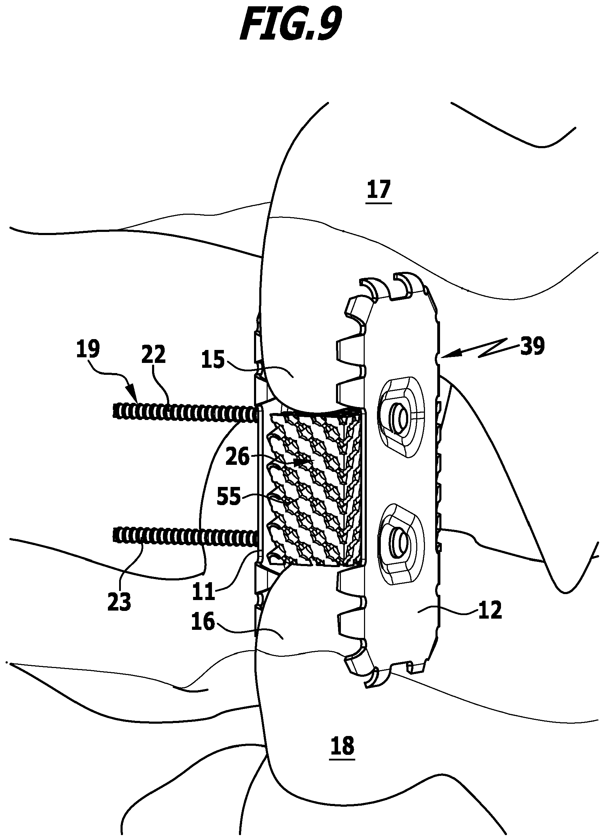

Fig. 9: shows the fixation system from Fig. 4, fixed to two spinous processes;
Fig. 10: shows a lateral-medial view of a plurality of vertebral bodies, the spinous processes of two vertebral bodies, in each case, being fixed by a sixth preferred embodiment of a fixation system according to the disclosure relative to one another;
Fig. 11: shows a schematic side view of a portion of an implantation tool during application on the fixation system from Fig. 10;
Fig. 12: shows a sectional view along the line 12-12 in Fig. 11;
Fig. 13: shows a sectional view along the line 13-13 in Fig. 12;
Fig. 14: shows a schematic sectional view of a portion of a further implantation tool, applied to the fixation system from Fig. 10;
Figs. 15A to 15C: show a perspective view of a seventh preferred embodiment of a fixation system according to the disclosure when being fixed to two spinous processes, a spacer element being introduced between two spinous processes at the beginning (Figs. 15A and 15B) and Fig. 15C showing the fixation system in the implantation position;
Fig. 16: shows a perspective view of an eighth preferred embodiment of a fixation system according to the disclosure when being fixed to two spinous processes;
Fig. 17: shows a perspective view of a ninth preferred embodiment of a fixation system according to the disclosure when being fixed to two spinous processes;
Fig. 18A and 18B: show a perspective view of a tenth preferred embodiment of a fixation system according to the disclosure, being a fixation system according to the invention, when being fixed to two spinous processes, a spacer element firstly being introduced between the spinous processes (Figs. 18A) and Fig. 18B showing the fixation system in the implantation position;
Fig. 19: shows a perspective view of an eleventh preferred embodiment of a fixation system according to the disclosure;
Fig. 20: shows a side view of an implantation tool when cooperating with the fixation system from Fig. 19;
Fig. 21: shows an enlarged partial view of the tool from Fig. 20 when cooperating with the fixation system from Fig. 19;
Fig. 22: shows a perspective view of a twelfth preferred embodiment of a fixation system according to the disclosure;
Figs. 23A and 23B: show respectively a schematic plan view and a schematic side view of a fixing element of one of the fixation systems mentioned above;
Figs. 24A and 24B: show respectively a schematic plan view and a schematic side view, partially sectional, of a further fixing element of one of the fixation systems mentioned above; and
Figs. 25A and 25B show a thirteenth preferred embodiment of a fixation system according to the disclosure in an insertion position (Fig. 25A) and in an implantation position (Fig. 25B).

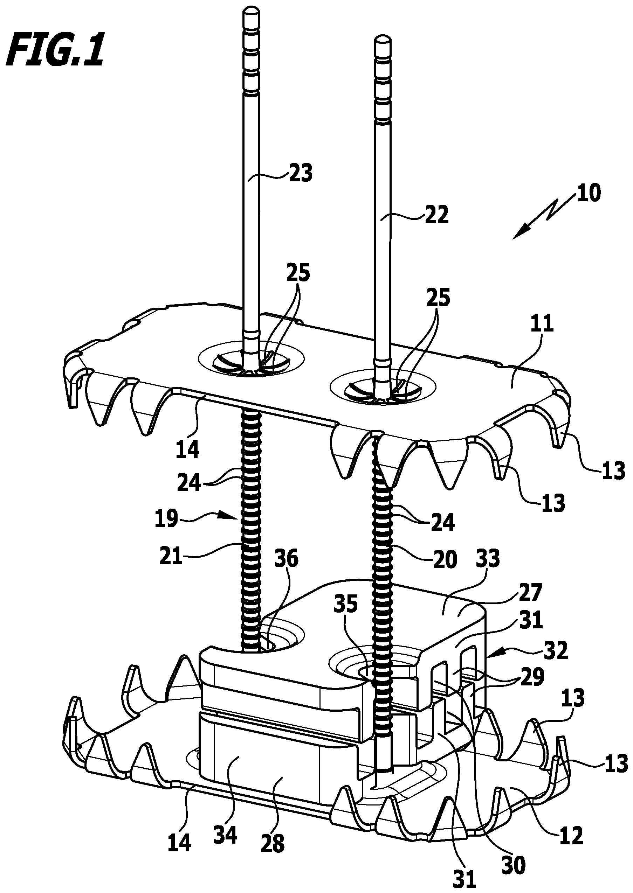

The first preferred embodiment of the fixation system 10 shown perspectively in Fig. 1 comprises a first, rectangular fixing element 11 and a second fixing element 12, which is of substantially identical configuration to the first fixing element 11. Both fixing elements, on their edges carry engagement members directed towards the respective other fixing element and substantially projecting transversely from the planar fixing elements, in the form of tooth-shaped projections 13. However, on their respective long sides, the two fixing elements 11, 12 have regions 14, which are free of projections 13 and which in each case extend over approximately a central third of the length of the fixing elements 11, 12.

By means of the projections 13, the fixing elements 11, 12 can penetrate into bone material, for example into spinous processes 15 and 16 of vertebral bodies 17 or 18, respectively. The vertebral bodies 17 and 18 may be human or animal vertebral bodies. "Vertebral body" in the present case designates both the vertebra as a whole and also the vertebral body in the narrower sense ("corpus vertebrae").

By penetrating into the spinous processes 15 and 16, the fixing elements 11 and 12 can secure them against a movement relative to one another in the cranial-caudal, dorso-ventral and lateral direction. The fixing elements 11, 12 are in this case applied laterally from different sides onto the spinous processes 15, 16, each of the fixing elements 11, 12 being able to penetrate into both spinous processes 15, 16 and the fixing elements being oriented with their longitudinal direction in the cranial-caudal direction.

The fixation system furthermore comprises a securing device 19, which is configured as a latching device and comprises two identical latching elements 20, 21. The latching elements 20, 21 are tie rods 22, 23 which are fixed to the fixing element 12, oriented perpendicular thereto and on which the fixing element 11 can be latched in a self-locking manner. For this purpose, the tie rods 22, 23 have peripheral ribs 24 into which resilient arm elements 25 on the fixing element 11 can latch.

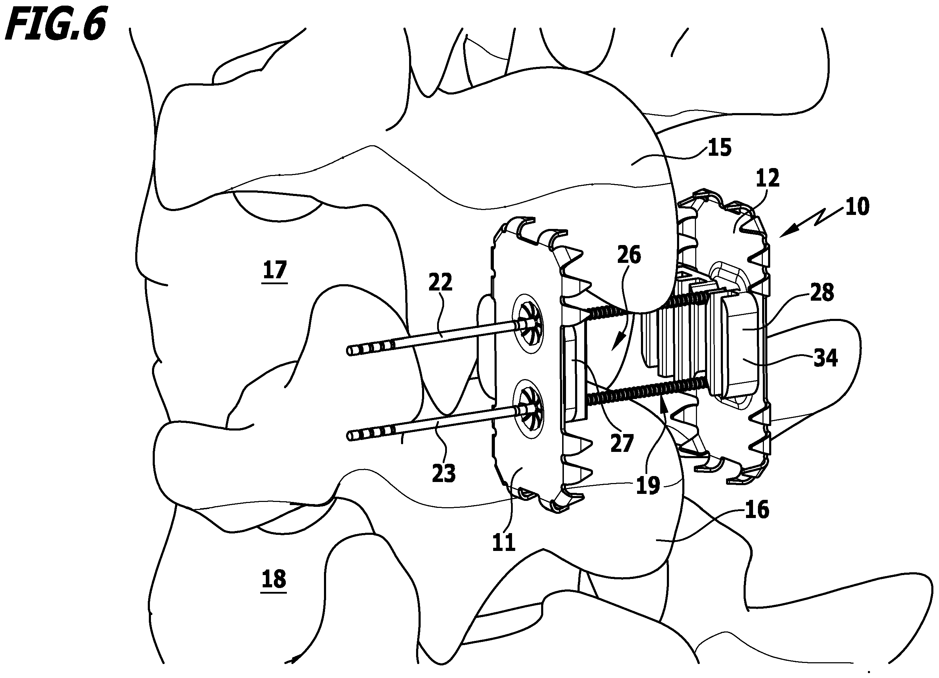

By means of the securing device 19, the fixing elements 12, 13 can be immovably fixed to the spinous processes 15, 16, in that one of the fixing elements 12, 13 is placed on the spinous processes 15, 16, with the tie rods 22, 23 engaging through an intervertebral space 26 from lateral to lateral. If the fixing elements 11, 12 are moved toward one another, the other one of the fixing elements 11, 12 also engages in the spinous processes 15, 16. A renewed removal of the fixing elements 11, 12 from one another is not possible as they are secured by means of the latching securing device 19 against a movement increasing their spacing from one another. A position of this type of the fixing elements 11, 12 relative to one another defines an implantation position, in which the fixing elements 11, 12 have a smaller spacing from one another than in an insertion position (Fig. 6), in which the fixation system 10 can be inserted into the body interior for implantation.

The fixation system 10, as described up to now, is a fixation system in the manner of the sternal closure device described in document DE 103 26 690 B4 and in the manner of the fixing device described in the document DE 10 2006 021 025 B3. Reference is expressly made hereby to the aforementioned documents. The disclosure also relates to a use of a sternal closure device identical to that or in the manner of that, as described in DE 103 26 690 B4, and to the use of a fixing device identical to or in the manner of the fixing device described in document DE 10 2006 021 025 B3 for fixing the spinous processes of adjacent vertebral bodies relative to one another with at least one spacer element being positioned in the intervertebral space between the spinous processes.

Furthermore, the fixation system 10 comprises two spacer elements 27 and 28, which are positioned between the fixing elements 11 and 12 and are inserted into the intervertebral space 26 during the use of the fixation system and are fixed therein by clamping. In the longitudinal direction of the fixing elements 11, 12, they adopt approximately the width of the free region 14. The spacer elements 27, 28 in each case have cranial-caudally extending ribs 29 and grooves 30, so the spacer elements 27 and 28 can positively engage in one another in the manner of combs, their spacing from one another being variable in the lateral-lateral direction, depending on to what extent they engage in one another. "Lateral-lateral" is to be understood as transverse to the cranial-caudal direction in the intervertebral region 26 substantially in the median plane, to a certain extent "lateral-medial-lateral" in the intervertebral region 26.

The spacer elements 27 and 28 in each case have a cranial contact face 31 for the spinous process 15, a caudal contact face, not shown in the drawing, for the spinous process 16, an anterior contact face 32 for the laminae arcus vertebrae of the vertebral bodies 17 and 18 and a lateral contact face 33 for the fixing elements 11 and 12. In the ventral direction they extend over the fixing elements 11, 12.

By means of the cranial contact face 31 and the caudal contact face, the spinous processes 15 and 16 can be supported in the cranial-caudal direction, the contact face 32 is used for stabilisation in the dorso-ventral direction of the vertebral bodies 17 and 18, and by means of the contact faces 33, the spacer elements 27, 28 are fixed in force-locking manner by clamping in the intervertebral space 26 by the fixing elements 11, 12. Overall, a well-defined position of the fixing elements 11, 12 relative to one another can be obtained. This makes possible a reliable stabilisation of the spinous processes 15 and 16 relative to one another.

The fixation system 10 furthermore proves to be usable in diverse ways, because the spacer elements 27 and 28, depending on the width of the intervertebral space 26 in the lateral-lateral direction, can engage to different extents with one another. This makes it possible to reliably support spinous processes of different dimensions against one another by means of the spacer elements 27, 28.

Formed posterior to the spacer elements 27 and 28, between the fixing elements 11, 12, is an intermediate space 34, which can, for example, be additionally filled with a bone material or bone replacement material when the fixation system 10 is applied.

Furthermore, the spacer elements 27, 28 have recesses 35 and 36, in which the tie rods 22 and 23 can engage. As a result, the spacer elements 27 and 28 are also fixed by clamping between the tie rods 22, 23 in the cranial-caudal direction. In the implantation position of the fixation system, they are particularly reliably fixed thereby in the intervertebral space 26.

Despite the versatility already mentioned, the fixation system can additionally be constructed in a modular manner; it can comprise fixing elements 11, 12 of different dimensions here as well as spacer elements 27 and 28 of different dimensions, which can be combined with one another as required. This reduces the quantity of material to be stored by the user and, nonetheless, the most varied requirements predetermined by different vertebral bodies can be individually taken into account.

The spacer elements 27, 28 may be rigid, they may be deformable, they may be resiliently deformable, they may be resorbable, they may be made from a metal, such as for example titanium, they may be made from a plastics material such as, for example, PEEK, they may consist of bone or other bone replacement material such as, for example, hydroxyapatite, they may be at least partially coated, for example with a Plasmapore coating and they may be osteointegrative. Further advantageous properties of the spacer elements 27, 28 are conceivable.

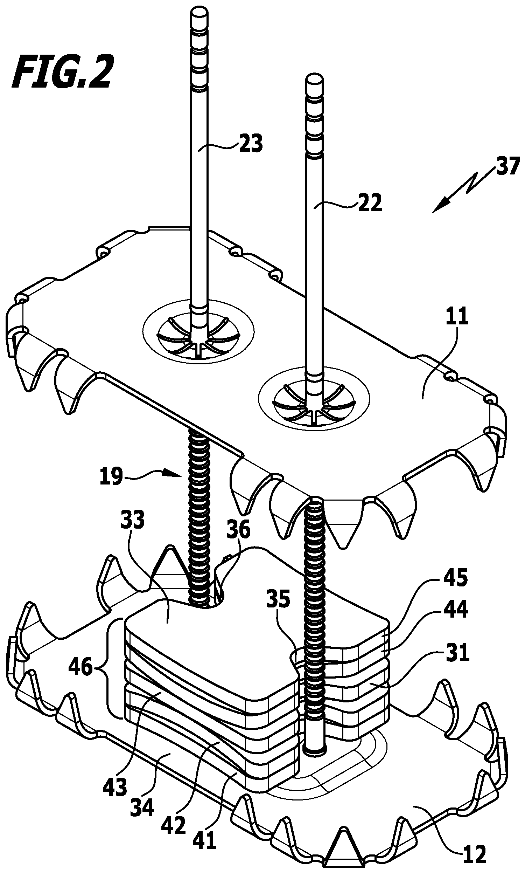

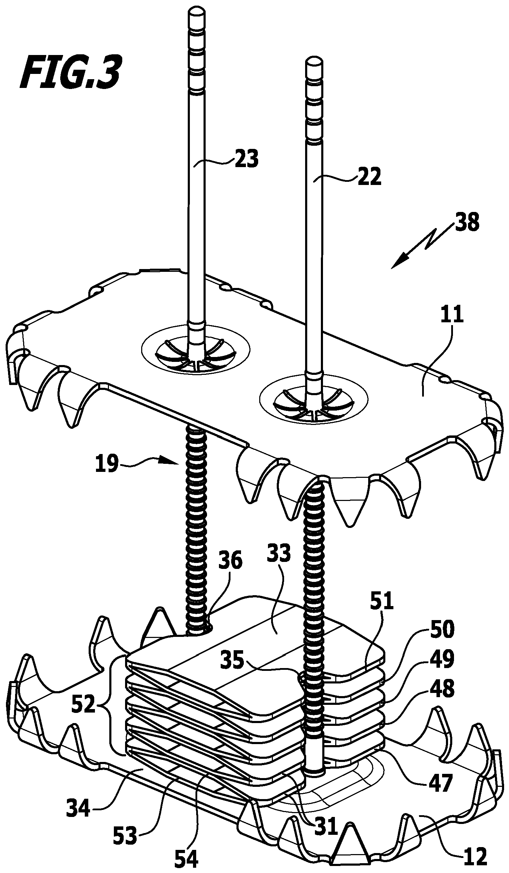

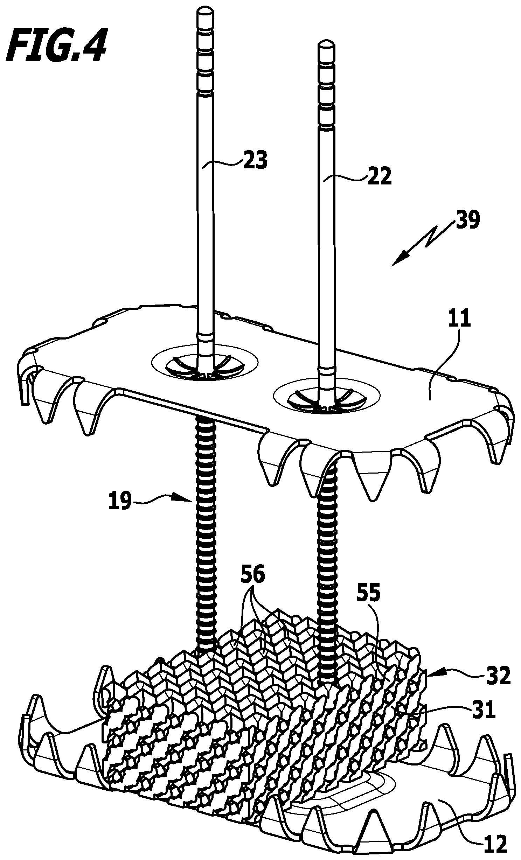

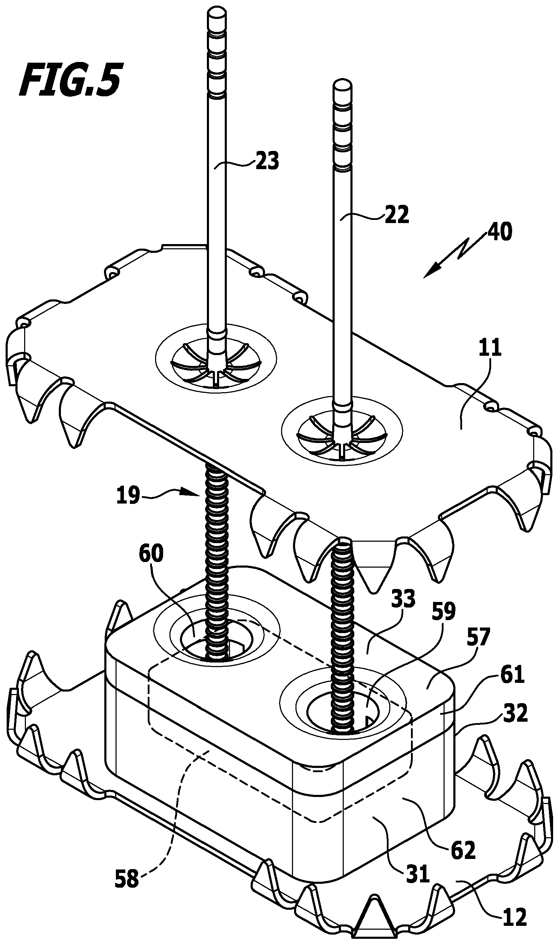

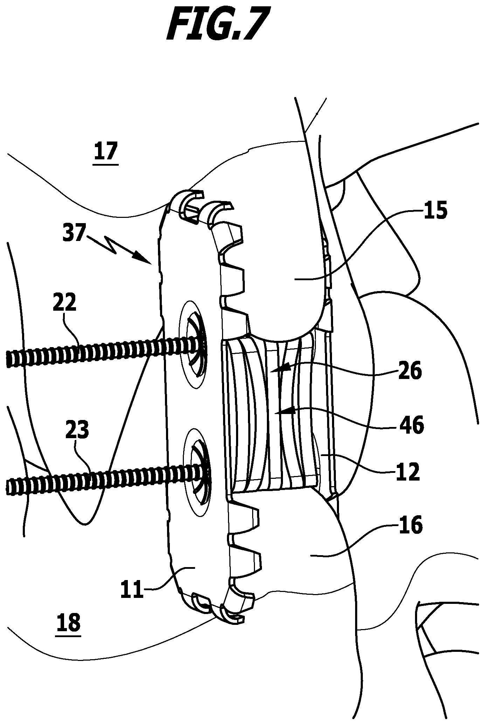

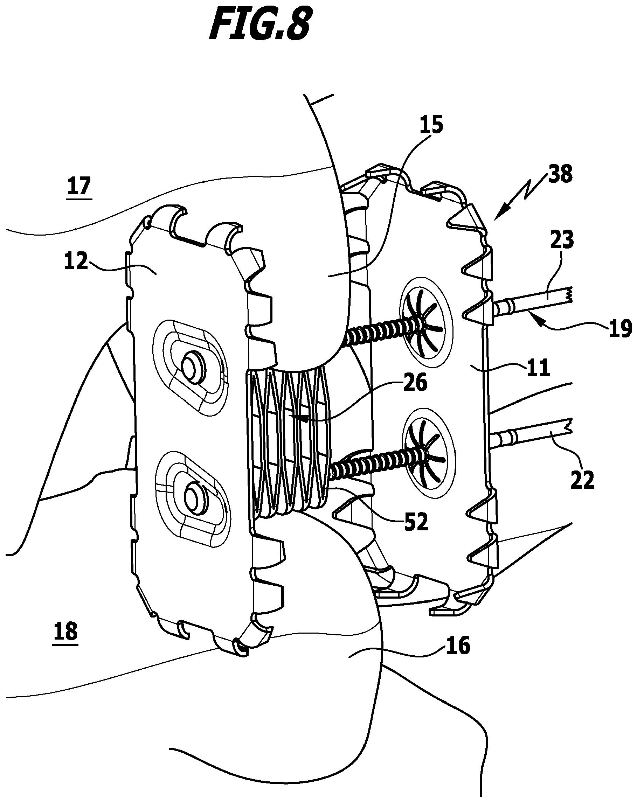

Figs. 2 to 5 show further preferred embodiments 37, 38, 39 or 40 respectively, of the fixation system according to the disclosure. Figs. 7 and 8 show the fixation systems 37 or 39 respectively, in each case in the implantation position fixed on the spinous processes 15, 16, and Fig. 8 shows the fixation system 38 in an insertion position, a fixing element 12 of the fixation system 38 already being engaged with the spinous processes 15, 16.

All the fixation systems 10, 37, 38, 39 and 40 use the same fixing elements 11 and 12 and the same securing device 19, and they only differ with respect to their spacer elements, so that reference will only be made to the spacer elements of the fixation systems 37 to 40 below and otherwise reference is made to the above statements on the fixation system 10.

Identical and identically-acting features or components of the spacer elements of the fixation systems 10 and 37 to 40 have the same reference numerals.

The fixation system 37 (Figs. 2 and 7) comprises a plurality of a total of five spacer elements 41, 42, 43, 44 and 45. The spacer elements 41 to 45 are in each case planar in a disc form, each being oriented approximately in a sagittal plane. In the lateral-lateral direction, adjacent spacer elements in the non-implanted state, abut against one another along contact lines. The spacer elements 41, 43 and 45 are planar, whereas the spacer elements 42 and 44 are convex or concave respectively, in the lateral-lateral direction.

The spacer elements 41 to 45 are preferably resiliently deformable, so that they form a resilient spacer package 46 in the lateral-lateral direction in their entirety. When transferring the fixing elements 11 and 12 into the implantation position (Fig. 7), the spacer package 46 can be compressed in the lateral-lateral direction. On the one hand, this provides the possibility of using the spacer package 46 in a large number of spinous processes 15, 16 of different dimensions and nevertheless allowing a well-defined relative position of the fixing elements 11, 12 and a reliable support of the spinous processes 15, 16. On the other hand, the spacer package 46 is reliably fixed relative to the fixing elements 11, 12, which it in each case acts upon with a medial-lateral force counteracting the clamping force of the fixing elements 11, 12.

The fixation system 37 can also be constructed in a modular manner, for example the number of spacer elements may be varied and selected depending on the size and type of spinous processes 15, 16. Furthermore, it is possible for the spacer elements 41 to 45 to project anteriorly beyond fixing elements 11, 12, so that they also form an anterior contact face 32 for the vertebral bodies 17, 18.

Provided in the fixation system 38 are five spacer elements 47, 48, 49, 50 and 51, which are formed identically and together form a spacer package 52. Each of the spacer elements 47 to 51 is a disc spring constructed from two segments 53 and 54 abutting against one another at the edge, which is deformable in the lateral-lateral direction and can flex. Each segment 53 of a spacer element rests flat on the segment 54 of an adjacent spacer element, and the spacer elements 47 to 51 are in each case oriented substantially in a sagittal plane.

Because of the resilient effect of each of the spacer elements 47 to 51, the spacer package 52 is also resilient in the lateral-lateral direction. The advantages mentioned as above in conjunction with the spacer package 46, to which reference is made, can thus already be achieved.

The fixation system 37 is also constructed in a modular manner. Thus, for example, the size of the spacer elements 47 to 51 or their number can be varied in order to allow a particularly individual adaptation of the fixation system 38 to the spinous processes 15, 16. A high level of diversity of the fixation system 38 as well is made possible purely because of the spacer package 52.

The fixation system 39 comprises a one-piece spacer element 55, which is lattice-shaped and can be made, in particular, from a metal such as, for example, titanium. Because of the lattice-shaped structure, the spacer element 55 is porous and therefore has particularly good osteointegrative properties. In particular, the spacer element 55 may be coated, for example with a Plasmapore coating.

Moreover, the spacer element 55 also has a cranial contact face 31, a caudal contact face and an anterior contact face 32. In the lateral direction, the spacer element 55 can rest virtually flat on the fixing elements 11, 12 by means of a large number of individual support points 56 and also be held, in the implantation position thereof in the intervertebral space 26 in a reliable manner by clamping.

The spacer element 55 is also fixed on the tie rods 22 and 23, for example they engage through said spacer element.

A modular configuration of the fixation system 39 is also possible, for example in that a plurality of spacer elements with the properties of the spacer element 55 is made available, in order to allow a particularly individual adaptation of the fixation system 39 to the spinous processes 15 and 16.

A spacer element 57, which is cuboidal and can be formed as a hollow body having an interior 58, is used in the fixation system 40. The interior 58 may be hollow, the spacer element 55 preferably having resiliently deformable properties. As a result, it forms a resilient buffer in the lateral-lateral direction between the fixing elements 11 and 12, so that the advantages which can be achieved by the spacer packages 46 and 52 can also be achieved with the spacer element 55. This is even possible when further spacer elements, for example, are received in the interior 58, for example spacer elements of the same type as the spacer elements 41 to 45 and/or of the same type as the spacer elements 47 to 51.

It may be provided that the spacer element 57 comprises two segments 61 and 62 that can be spaced relative to one another in order to have access to the interior 58. A particularly individual adaptation to the spinous processes 15, 16 can thereby be implemented, for example, in a modular configuration of the fixation system 40. For example, depending on requirement, one or more spacer elements can be inserted into the interior 58.

Furthermore, openings 59 and 60 for the tie rods 22 and 23 in the spacer element 57 are formed to allow particularly reliable fixing of the spacer element 57 in the implantation position of the fixation system 40. Moreover, the spacer element 57 has a cranial contact face 31 and a caudal contact face as well as respective lateral contact faces 33 for the fixing elements 11 and 12.

The spacer elements 41 to 45, 47 to 51 and 57 can have all the properties, which have already been mentioned above in conjunction with the spacer elements 27 and 28 of the fixation system 10.

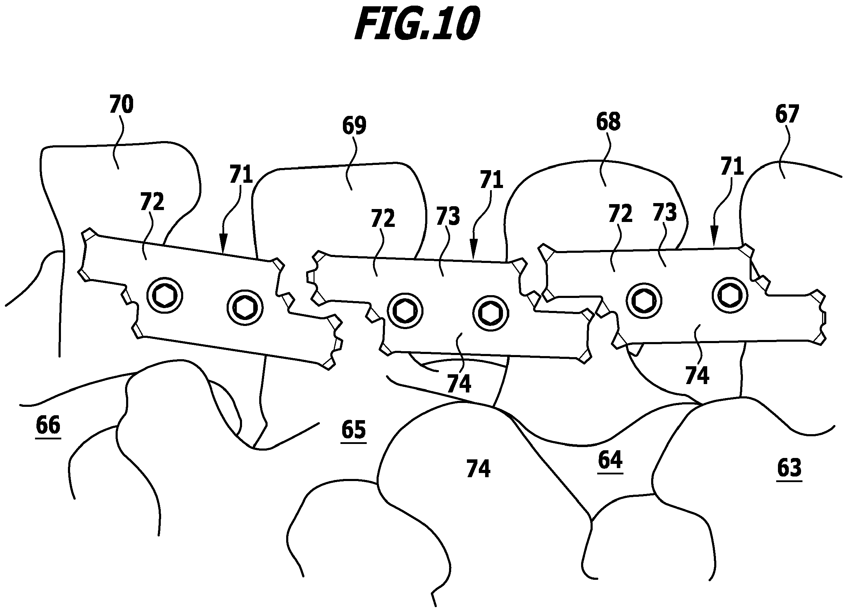

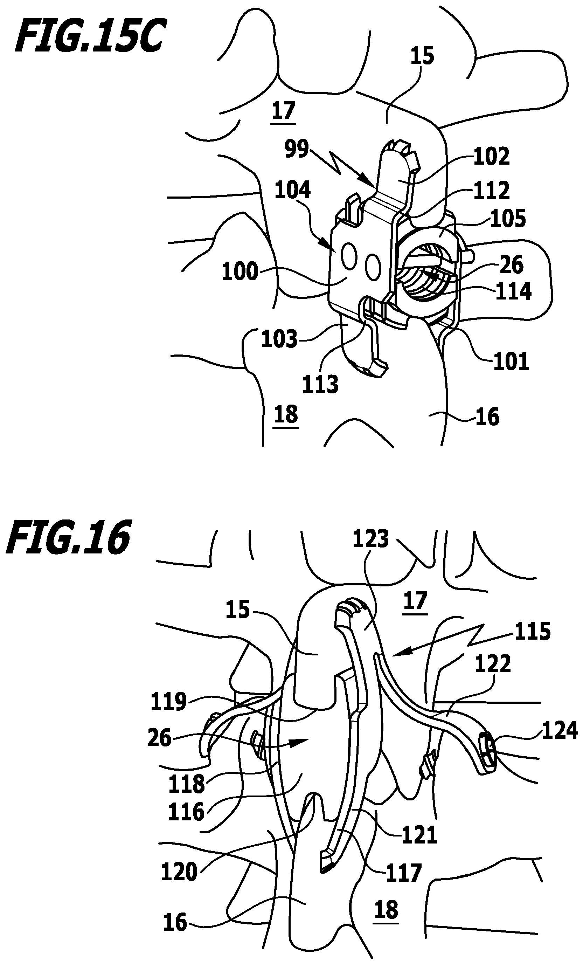

Fig. 10 shows in a lateral-medial view, four vertebral bodies 63, 64, 65 and 66 with spinous processes 67, 68, 69 and 70, respectively. One fixation system 71 in each case of a sixth preferred embodiment fixes the spinous processes 67 and 68, 68 and 69 as well as 69 and 70 to one another. The fixation system 71 differs from the fixation system 10 in that its fixing elements, of which only one fixing element 72 is shown, do not have a rectangular configuration. The fixing element 72 instead comprises two, in each case rectangular portions 73 and 74, which are offset relative to one another in the cranial-caudal direction. The offset is approximately one third of the length of the fixing element 72, but it could also be greater, for example about 40% or about half of the length of the fixing element 72 (see, for example, the variants in Figs. 15C, 17, 22 and 23A to 24B). The fixing element 72 thus receives an overall step-like construction. This is advantageous if a plurality of spinous processes 67 to 70 is to be stabilised. In this case, as shown in Fig. 10, projecting regions of the portions 73 and 74 of a fixing element 72 can in each case project into set-back regions of the portions 73 and 74 of an adjacent fixing element 72.

Otherwise, the fixation system 71 is formed like the fixation system 10, so reference can be made to the above statements. It is also possible for the fixing elements 72 to be used instead of the fixing elements 11 and 12 in each of the fixation systems 37 to 40.

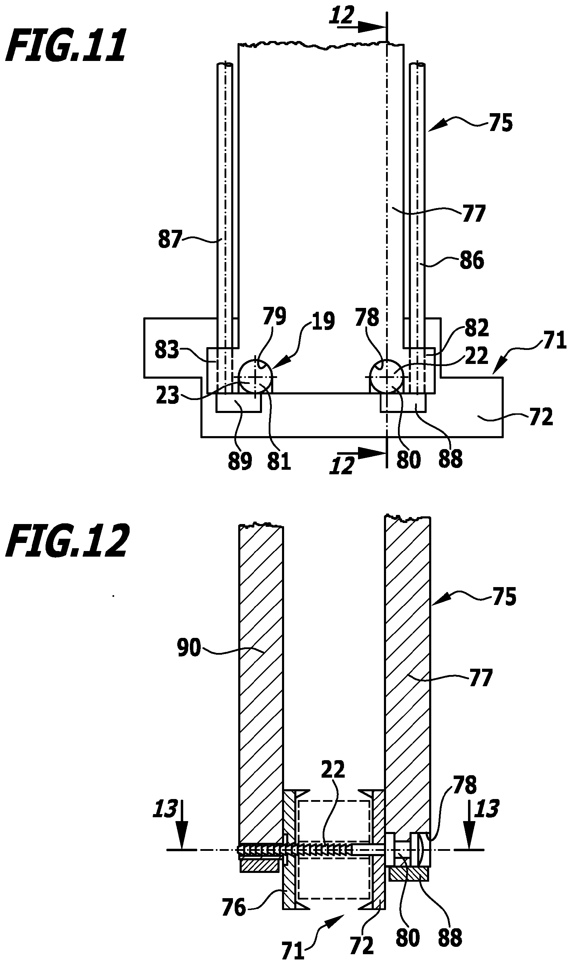

To implant the fixation systems 10, 37 to 40 and 71, a tool 75 shown only schematically and in portions in Figs. 11 to 13 can be used. It is shown cooperating with the fixation system 71, wherein apart from the fixing element 72, a fixing element 76 formed mirror-inverted thereto, the tie rods 22 and 23 of the securing device 19 and a spacer element of the fixation system 71 are also shown.

The tool 75 comprises a first tool part 77 with end, cavity-shaped recesses 78 and 79 for end portions 80 or 81 of the tie rods 22 or 23, respectively. Adjacent to the recesses 78 and 79, the tool part 77 has exterior projections 82 or 83 with openings 84 or 85, respectively. Guided through the openings 84 and 85 are angled fixing parts 86 or 87, respectively, which run parallel to the tool part 77 and, at a right angle to the longitudinal direction thereof, comprise holding portions 88 or 89. The holding portions 88 and 89 can engage below the end portions 80 and 81. The fixing parts 86 and 87 are in each case rotatably mounted in the openings 84 and 85 about an axis defined by the longitudinal direction thereof.

A second tool part 90 of the tool 75 is substantially identically formed to the first tool part 77 and is used to handle the fixing element 76.

To use the tool 75, the end portions 80 and 81 can be introduced into the recesses 78 or 79 and the fixing parts 86 and 87 can be rotated relative to the tool part 77 in such a way that the holding portions 88 and 89 positively fix the end portions 80 and 81 in the recesses 78 or 79, respectively (Fig. 12).

The same applies to the ends of the tie rods 22 or 23, which oppose the end portions 80 and 81, respectively, and can also be held by means of the tool part 90 and fixing parts formed corresponding to the fixing parts 86 and 87. The fixation system 71 is thus held on the tool 75 and can be introduced therewith into the body. By pivoting the tool parts 77 and 90 relative to one another about a pivot axis oriented perpendicular to the longitudinal direction of the tool parts 77 and 90 and perpendicular to the longitudinal direction of the tie rods 22 and 23, the fixing elements 72 and 76 can be moved closer to one another and thereby transferred into an implantation position. A displacement of the tool parts 77 and 90 relative to one another along the tie rods 22 and 23 also allows a transfer of the fixing elements 72 and 76 into the implantation position.

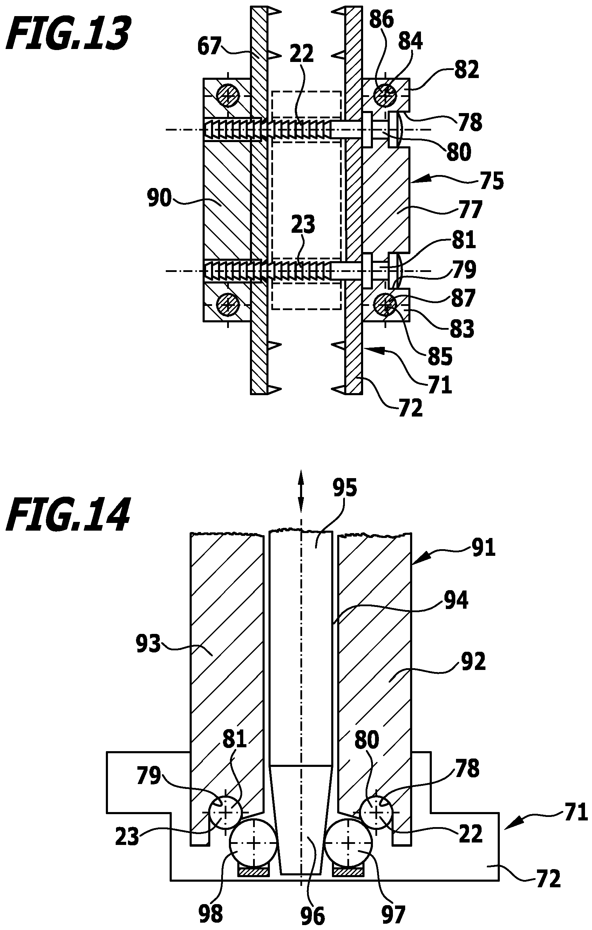

Fig. 14 schematically shows a further tool 91, in which the tool part 77 is to a certain extent divided in the transverse direction into two tool parts 92 and 93, between which an intermediate space 94 is formed. An actuator 95 is displaceably mounted in the intermediate space 94 in the longitudinal direction of the tool 91. The actuator 95 is tapered conically at the end. If the actuator 95 is displaced relative to the tool parts 92 and 93, its conically tapered end portion 96 can displace blocking elements 97 and 98 in the form of balls transverse to the displacement direction, so that the end portions 80 and 81 are fixed in the recesses 78 or 79, respectively. The tool 91 in this case, manages without the fixing parts 86 and 87.

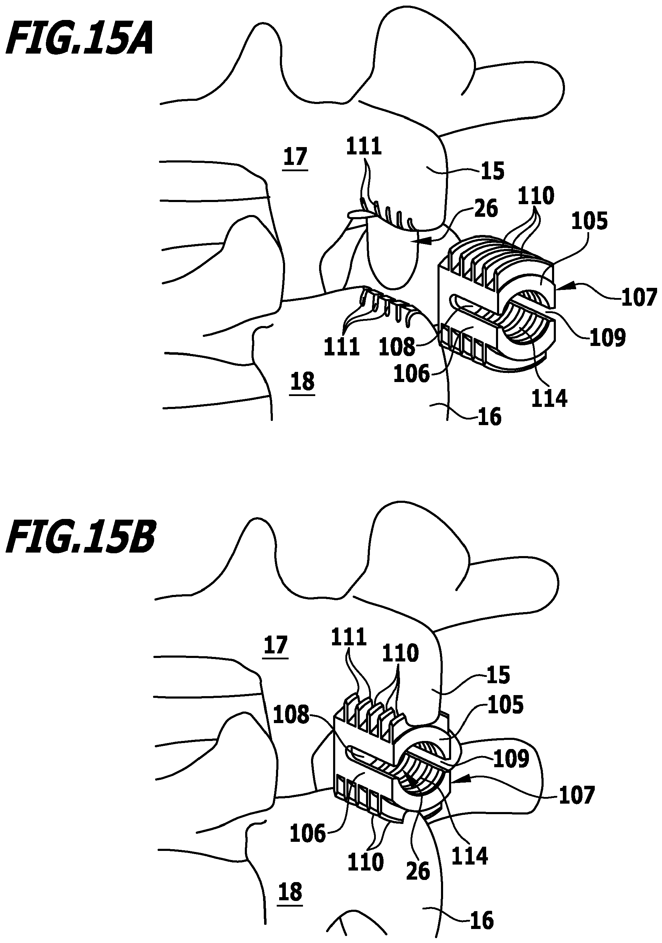

Figs. 15A to 15C partially or completely show a seventh preferred embodiment of a fixation system according to the disclosure having the reference numeral 99. The fixation system 99 comprises, similar to the fixation system 71, fixing elements 100 and 101, which are mirror inverted with relative to one another, each with rectangular portions 102 and 103 offset with respect to one another in the cranial-caudal direction. The portions 102 and 103 approximately half overlap one another in each case. Furthermore, a securing device 104 in the manner of the securing device 19 of the fixation system 10 is provided. The above statements are referred to in this regard.

The fixation system 99 also comprises a one-piece spacer element 105 formed, for example, from plastics material or metal. The spacer element 105, with regard to the basic shape, has the shape of a hollow cylinder, from which axially extending arcuate segments have been removed, in each case, on mutually opposing lateral sides. As a result, planar lateral contact faces 106 or 107 facing the fixing elements 100 and 101, respectively, are formed on the spacer element 105. A slot-shaped recess 108 or 109 extending in an axially parallel manner is formed into each of the contact faces 106 and 107, respectively.

Between the contact faces 106 and 107, a plurality of cutting edges 110 sharply tapered in the radial direction and spaced apart from one another in the dorso-ventral direction run on the outside of the spacer element 105. The cutting edges 110 may be segments of a spiral cutting edge extending over the surface of the spacer element 105, to a certain extent individual threads of a screw formed by the spacer element 105, which are interrupted in each case on the contact faces 106 and 107. It is also conceivable for the cutting edges 110 not to arise from a spiral of this type, but to in each case be arranged in a plane oriented perpendicular to the dorso-ventral direction. This is the case in the embodiment shown in Figs. 15A to 15C, in which the cranially oriented and the caudally oriented cutting edges are offset relative to one another in the dorso-ventral direction, in other words "with a gap". A caudal contact face, not visible in detail, and a cranial contact face, also not visible in detail, for the spinous process 16 or 15 are formed on the shell of the hollow-cylindrical spacer element 105 between the cutting edges 110.

When implanting the fixation system 99, the procedure can be as follows:
Firstly, the intervertebral space 26 is prepared, for example with a suitable thread cutter, in order to form slot-shaped recesses 111 to receive the cutting edges 110 on the spinous processes 15 and 16. The spacer element 105 can then be screwed into the spinous processes 15 and 16 if it has screw-shaped cutting edges 110. If this is not the case as in the variant shown, the spacer element 105 can be introduced into the intervertebral space 26 rotated through 90° compared to the configuration shown in Figs. 15A to 15C and rotated therein through 90° such that the cutting edges 110 cooperate with the recesses 111 (Fig. 15B). In the cranial-caudal direction, the spinous processes 15 and 16 are thus resiliently supported relative to one another, the spacer element 105 being particularly reliably fixed on the spinous processes 15 and 16.

The fixing elements 100 and 101 are then placed laterally on the spinous processes 15 and 16 and transferred to the implantation position, being secured therein by means of the securing device 104 (Fig. 15C). In the lateral-lateral direction, the spacer element 105 is dimensioned such that the fixing elements 100 and 101 in their regions engaged with the spinous processes 15 and 16 are slightly deformable in the lateral-medial direction. For example, this can be seen from the bending edges 112 and 113 of the fixing element 100. As a result, the fixing elements 100 and 101 are additionally prestressed relative to one another so that the fixation system 99 is reliably fixed.

It can be provided in the hitherto mentioned further preferred embodiments of a fixation system according to the disclosure that the respective fixing elements, as with the fixing elements 100 and 101 of the fixation system 99, are also to be deformed in the lateral-medial direction and, in particular, to be bent, for example in a spacer element or spacer package projecting out of the intervertebral space in the lateral direction.

It may further be provided in the fixation system 99 that the hollow space 114 enclosed by the spacer element 105 is filled with bone or bone replacement material in order to achieve still better cranial-caudal support properties. This may, for example, also be provided if, instead of the spacer element 105, a hollow screw is used that is screwed to the spinous processes 15 and 16.

The spacer element 105, in manner corresponding to the spacer elements 27 and 28 of the fixation system 10, can have a large number of different properties already mentioned above, to which reference is hereby made in order to avoid repetitions. These properties can also be fulfilled in the spacer elements of the fixation systems to be described below, so that these properties will not be listed again below either and reference is made to the above statements.

Likewise, the fixation system 99 and the fixation systems still to be described below, as with the fixation system 10, can be modularly constructed. Depending on the type and size of the spinous processes 15 and 16 to be connected to one another, the operator can be provided with a set of surgical instruments which comprise a plurality of, for example, spacer elements of different sizes formed in the same manner as the spacer element 105, and the same applies to the fixing elements 100 and 101. As required, for example according to the type of vertebral bodies 17 and 18, and also the size of the patient or the like, the operator can take the fixing elements best suited for the respective use and the spacer element(s) from the instruments and implant them in combination with one another.

A variant of the spacer element 105 is formed as a coil spring, which can be inserted in the dorso-ventral direction into the intervertebral space 27 and projects laterally out of it in each case. By transferring the fixing elements 100 and 101 into the implantation position, this coil spring can be compressed in the lateral-lateral direction and thereby a cranial-caudal prestressing and therefore support between the spinous processes 15 and 16 can be achieved (not shown).

Fig. 16 shows an eighth preferred embodiment of a fixation system according to the disclosure which has the reference numeral 115. The fixation system 115 comprises a one-piece spacer element 116 and a pair of fixing elements 117 and 118 formed mirror-inverted with respect to one another and furthermore also a securing device, not shown in the drawing, in the manner of the securing device 19.

The spacer element 116 forms a groove-shaped cranial receiver 119 for the spinous process 15 with a corresponding contact face in the cranial direction and, furthermore, an also groove-shaped receiver 120 for the spinous process 16 with a corresponding contact face in the caudal direction. In the lateral-lateral direction, the spacer element 116 projects out of the intervertebral space 26.

The fixing elements 117 and 118 are deformable and, in particular, bendable. They in each case comprise two portions 121 and 122 extending in the longitudinal direction. The portions 121 and 122 are each in the form of a strip. At the cranial end of the fixing element, the portions 121 and 122 are connected to one another by a bridge 123, to a certain extent the "head" of the fixing element 117. The portion 121 is arranged posterior with respect to the portion 122, and it runs from the spinous process 15 laterally over the spacer element 116 to the spinous process 16 laterally, with which it engages, in each case, with it laterally fixing the spacer element 116 by clamping.

In contrast to this, the portion 122, proceeding from the bridge 123, is further bent over in the lateral direction, so that it extends both over the processus articularis inferior of the vertebral body 17 and also the processus articularis superior of the vertebral body 18. In the implantation position of the fixation system 115, the portion 122 is connected to the processus articularis superior of the vertebral body 18, for example by means of an anchoring member 124 in the form of a screw, which can also engage in the processus articularis inferior of the vertebral body 17. The same applies in a mirror-inverted manner to the fixing element 118. This allows the vertebral bodies 17 and 18 to be particularly reliably fixed to one another.

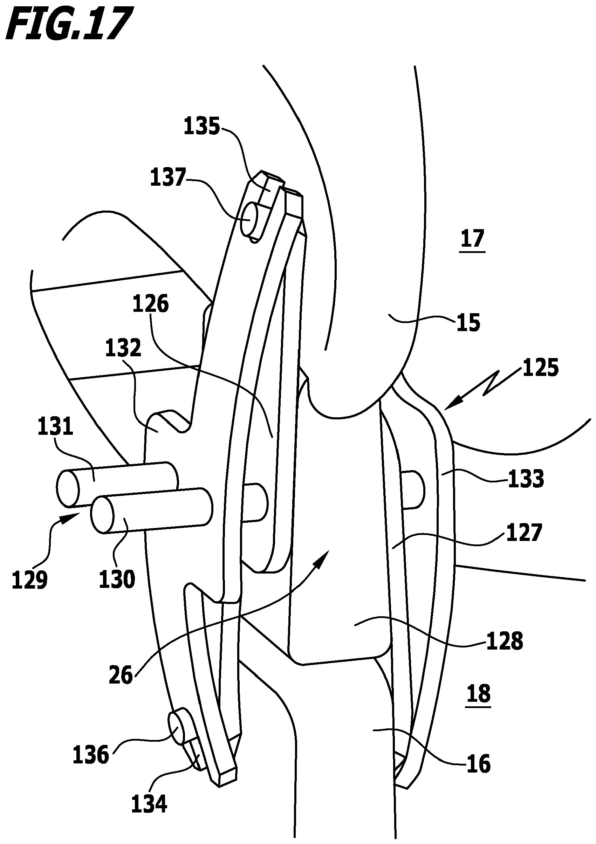

Fig. 17 shows a ninth preferred embodiment of a fixation system according to the disclosure and designated by the reference numeral 125, which is shown in the implantation position. The fixation system 125 comprises fixing elements 126 and 127, which are formed substantially identically to the fixing element 72, and a spacer element 128 fixed between them by clamping, substantially identically to the spacer element 116. The fixation system 125 further comprises a schematically shown securing device 129 with two securing elements in the form of latching tie rods 130 and 131.

Spring elements 132 and 133 cover the fixing elements 126 or 127 laterally, and they are formed in a similar manner to these from two portions offset relative to one another in the caudal-cranial direction. The spring elements 132 and 133, caudally and cranially at the end, have respective recesses 134 and 135, respectively, into which projections 136 arranged at the end side on the fixing elements 126 and 127 can engage from medial to lateral. The length of the spring elements 132 and 133 in the caudal-cranial direction is greater than that of the fixing elements 126 or 127, respectively, and they are further resiliently deformable. As a result, a preload, which can be transmitted to the spinous processes 15 and 16 for improved fixing of the fixation system 125, can be exerted by the spring elements 132 and 133 on the fixing elements 126 and 127 both in the lateral and in the cranial-caudal direction.

So that the spring elements 132 and 133 do not give way laterally, they can also be fixed relative to one another by means of the securing device 129, in particular, the tie rods 130 and 131 can be fixed to the spring element 133, this is not shown in the drawings. They engage through the fixing element 127, the spacer element 128, the fixing element 126 and the spring element 132, which can be fixed to them by latching relative to the spring element 133.

The fixing elements of all the fixation systems can also be connected to one another to form a frame, for example made of metal. A frame of this type can be used to incorporate bone or a bone replacement material in the intervertebral space 26, in addition to the spacer element being used in each case. For example, in the fixation system 125, the fixing elements 126 and 127 can be connected to one another posterior to the spinous processes 15 and 16 and together form a frame.

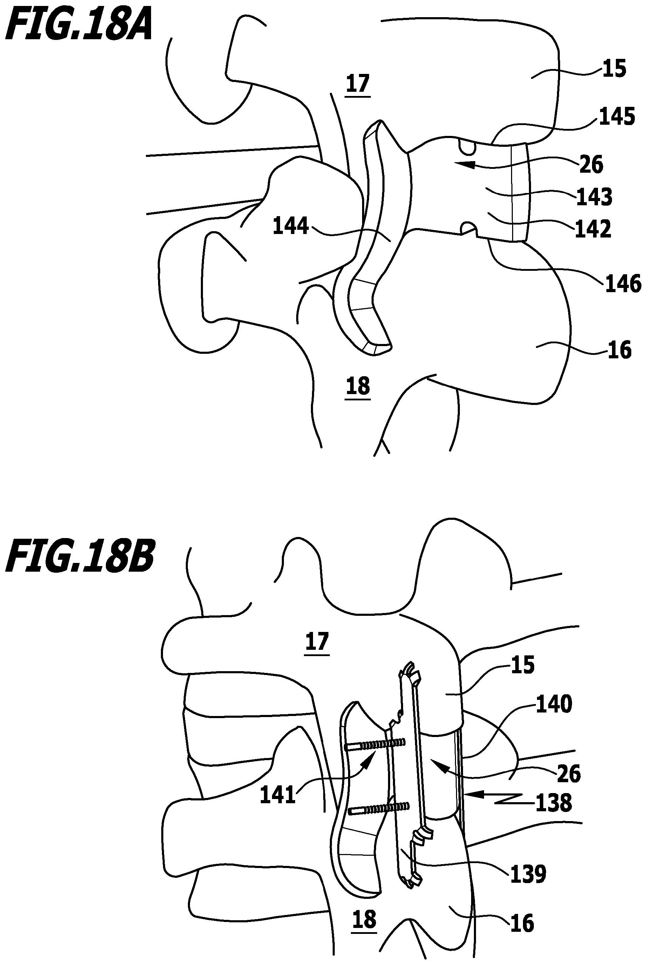

Figs. 18A and 18B show a tenth preferred embodiment of a fixation system according to the disclosure, being a fixation system in accordance with the invention, designated by the reference numeral 138. The fixation system 138 comprises fixing elements 139 and 140, which are substantially identically configured to the fixing element 72, a securing device 141 of the same type as the securing device 19 and a spacer element 142, which can, in particular, be formed in one piece and is constructed in substantially three portions, of which only portions 143 and 144 are shown in the drawing.

The first portion 143 is inserted in shape-locking manner into the intervertebral space 26 between the spinous processes 15 and 16, with it forming a cranial contact face 145 and a caudal contact face 146 for these.

The third portion, not shown, of the spacer elements 142 is mirror-inverted with respect to the second portion 144, so only the second portion 144 will be dealt with below. The second portion 144 is arranged anterior to the first portion 143, so a shape-locking connection is also achieved in the dorso-ventral direction between the spacer element 142 and the vertebral bodies 17 and 18. In particular, the second portion 144 is in the form of a strip and projects laterally from the first portion 143, in a way, as a "wing" thereof, and has an approximately sinusoidal profile in the lateral-medial transverse sectional direction. The second portion 144 can therefore rest flat on the laminae arcus vertebrae both of the vertebral body 17 and the vertebral body 18 and bring about a reliable dorso-ventral fixing.

In the cranial-caudal direction, the second portion 144 can, for example, extend from the processus articularis inferior of the vertebral body 17 to the processus articularis inferior of the vertebral body 18. It is even conceivable for the second portion 144 to extend in the cranial direction up to the processus articularis superior of the vertebral body 17. In a plan view from the dorsal direction, the spacer element 142 thereby receives approximately the profile of a large H.

When adapting the spacer element 142, it may be advantageous to prepare the laminae arcus vertebrae of the vertebral bodies 17 and 18 in advance, for example by punching. This provides the possibility of further increasing the shape-locking connection between the spacer element 142 and the vertebral bodies 17 and 18. If the spacer element is osteointegrative, the adhesion is thereby improved.

In the implantation position, the fixing elements 139 and 140 can laterally overlap the spacer element 142 in the manner already mentioned and be secured to one another by means of the securing device 141 (Fig. 18B).

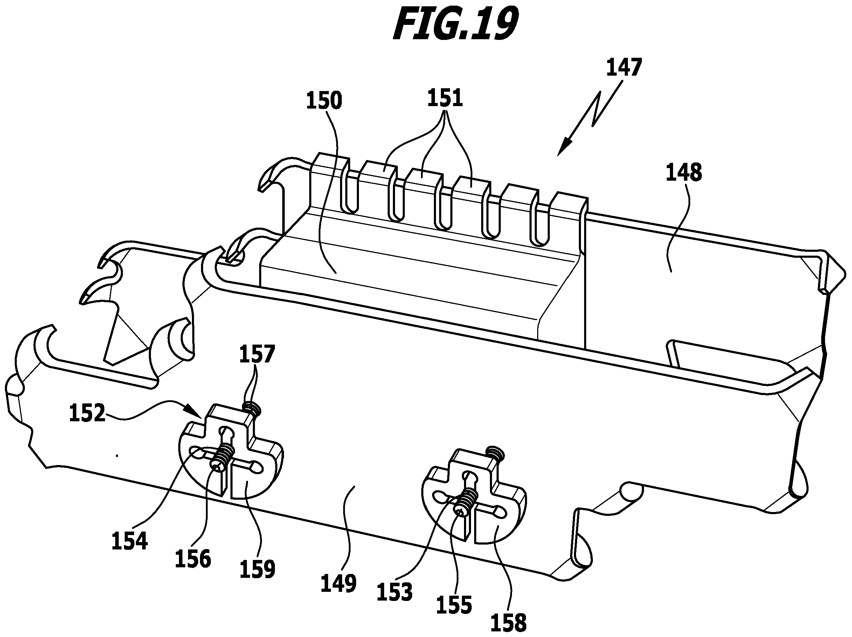

An eleventh preferred embodiment of a fixation system according to the disclosure is perspectively shown in Fig. 19 and has the reference numeral 147. It comprises fixing elements 148 and 149 in the manner of the fixing element 72 and a spacer element 150, which can be latched by means of latching arm elements 151 on at least one of the fixing elements 148, 149.

The fixation system 147 further comprises a securing device 152 for securing in the implantation position, which, is substantially identical to the securing device 19 of the fixation system 10. Accordingly, the securing device 152 comprises latching elements 153 and 154 in the form of tie rods 155 or 156, each with peripheral ribs 157. The tie rods 155, 156 are fixed to the fixing element 148.

For cooperation with the tie rods 155 and 156, the securing device 152 has locking members in the form of latching discs 158 or 159, respectively. The latching discs 158, 159 are slotted cross-wise and can be latched onto the tie rods 155, 156. In contrast to this, a latching of the fixing element 149 to the tie rods 155, 156 is not provided. This is nevertheless also possible.

At least the side of the latching discs 158, 159 facing the fixing element 149 can be concavely curved and thereby, for example, have a spherical contour. In practice, the latching disc 158, 159 can be oriented in an improved manner relative to the tie rod 155, 156 when the fixing element 159 tilts because of the anatomic circumstances of the spinous processes 15 and 16 and adopts an oblique orientation with respect to the fixing element 148.

Although the securing device 152 was initially described in the fixation system 147, it is obviously possible for the securing device 152 to be used instead of the respective securing device of each of the above-described fixation systems. It is furthermore conceivable for the spacer element 150 to be used instead of the respective spacer element(s) in one of the fixation systems mentioned above.

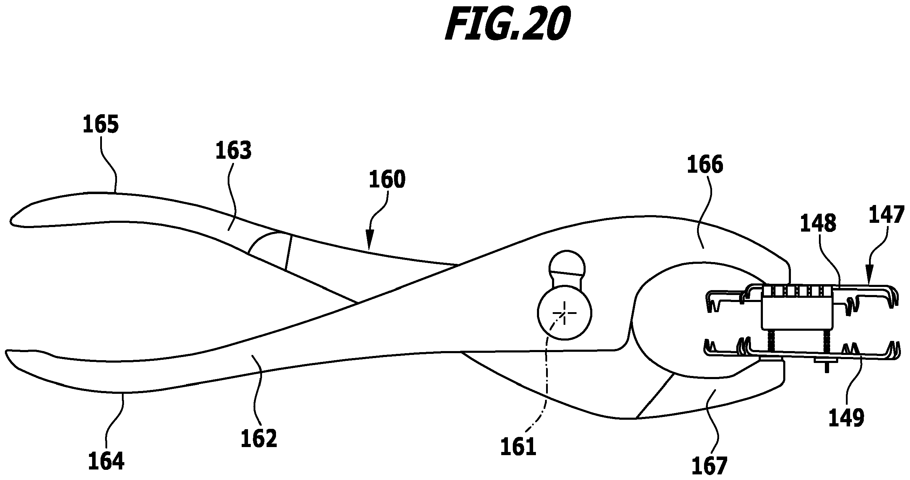

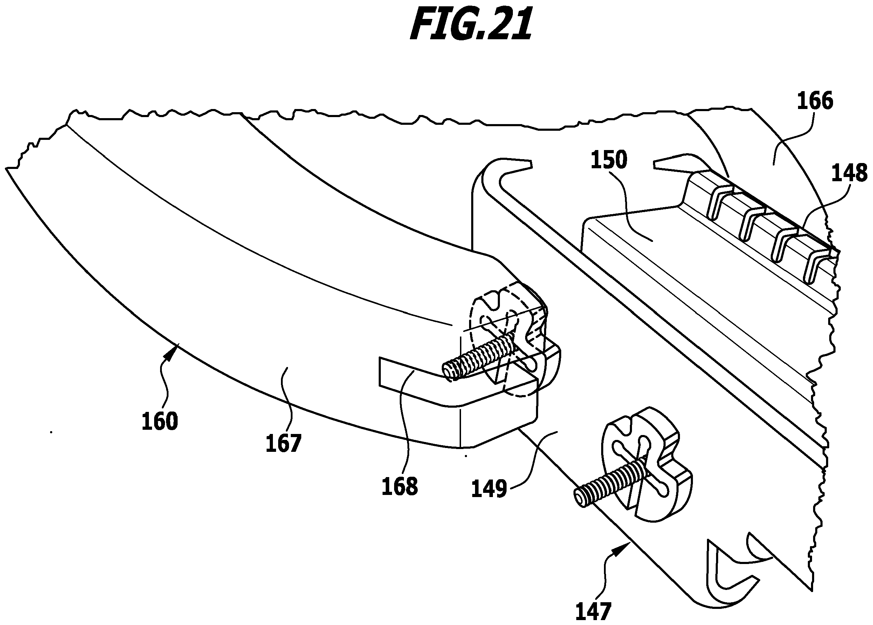

Figs. 20 and 21 show a further tool 160 for the implantation of one of the above-described fixation systems. The fixation systems, shown in Figs. 20 and 21 using the example of the fixation system 147, can be transferred by means of the tool 160 from the insertion position into the implantation position.

The tool 160 comprises two tool parts 162 and 163, which can be pivoted about a pivot axis 161 relative to one another, of which each has a handle element 164 or 165 and a jaw part 166 or 167, respectively. A groove-shaped recess 168 running transverse to the longitudinal direction of the tool 160 is formed at least on the jaw part 167 at the end. A comparable recess can also be formed at the end on the jaw part 166.

To transfer the fixation system 147 from the insertion position into the implantation position, the jaw part 166 can be placed on the fixing element 148 and the jaw part 167 can be placed on one of the latching discs 158, 159, the tie rod 155 or 156 associated with these latching discs 158, 159, respectively, in each case being able to engage in the recess 168. If the tool parts 162, 163 are pivoted relative to one another about the pivot axis 161 while moving the jaw parts 166, 167 towards each other, the respective tie rod 155, 156 can be guided through the recess 168 - optionally through the recess also formed on the mouth part 166 - for the reliable transfer of the fixation system 147 into the implantation position.

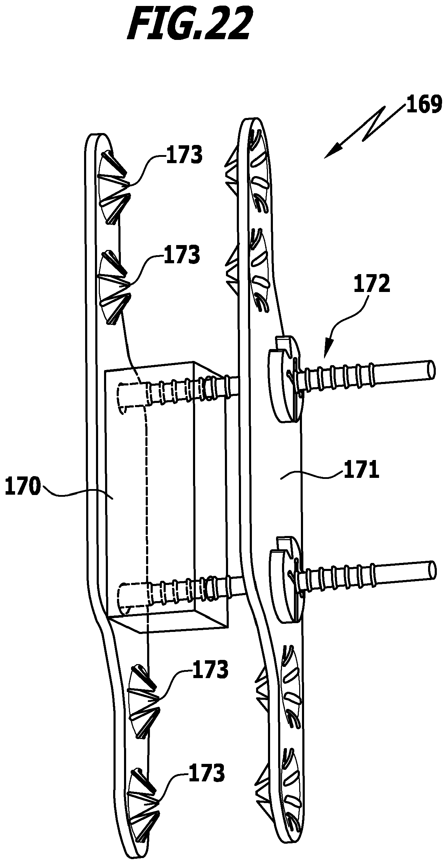

A twelfth preferred embodiment of a fixation system according to the disclosure is partially shown in Fig. 22 with a spacer element schematically depicted and has the reference numeral 169 therein. It comprises fixing elements 170, 171 with the shape of the fixing element 72 and a securing device 172 of the same type as the securing device 152.

The special feature of the fixation system 169 is that engagement members for anchoring the fixing elements 170, 171 to the spinous processes 15, 16 are not projections arranged at the edge of the fixing elements 170, 171, as in the fixation systems mentioned until now. Instead, projections 173 are arranged in the face of the fixing elements 170, 171, and they to a certain extent can be "extended" from the planes defined thereby, in each case.

For example, the fixing element 170 comprises four centres of projections 173, which, during insertion of the fixation system 169, can be arranged in the plane defined by the fixing element 170 or can adopt a small acute angle relative to this plane. This is shown in Fig. 22, for example. Arranged in a circle on each of the four centres of projections are projections 173, which are tapered and form engagement lugs to engage in the spinous processes 15, 16. By acting upon the projections 173 with a force directed onto the respective other one of the fixing elements, the projections 173 can be transferred to a larger angle with respect to the plane defined by the fixing element 170. If the fixing element is manufactured from metal, the projections 173 are, for example, bent over, perhaps with crimping pliers.

If the projections 173 in an engagement position of this type adopt a larger angle with respect to the fixing element 170, they can be anchored on the spinous processes 15, 16 in an improved manner. If the projections 173 adopt the insertion position, in which they are arranged in the plane defined by the fixing element 170 or only adopt an acute angle relative to it, the fixation system 169 can be inserted into the intervertebral space 26 in a simpler manner. In addition, a risk of injury to the patient can be reduced as the projections 173 can be extended not until during the final transfer of the fixation system 169 into the implantation position.

It can be provided that the further fixation systems described above comprise engagement members in the manner of the projections 173, in addition to the projections 13 or instead of the projections 13.

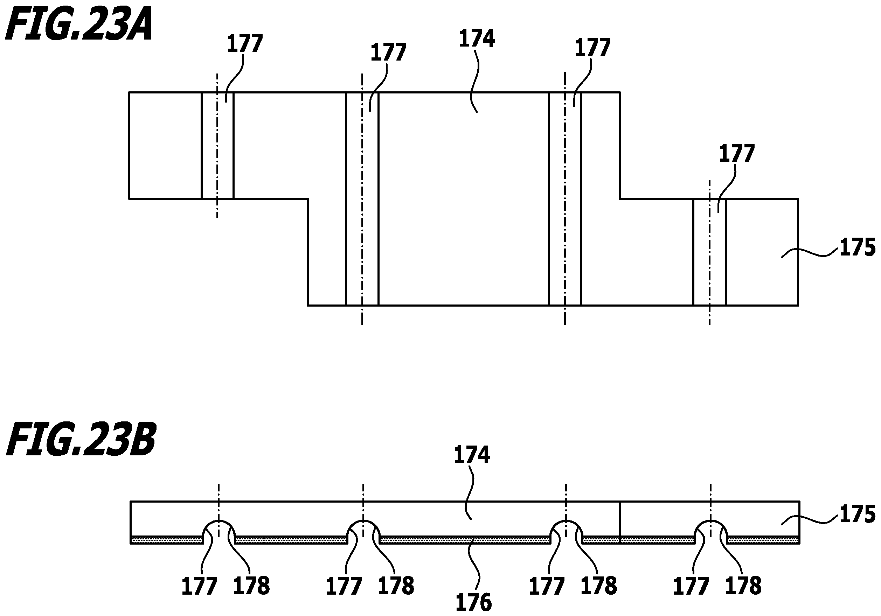

Figs. 23A and 23B show a plan view and a side view respectively of a fixing element 174, as can be used in each of the fixation systems described here. The fixing element 174 has a carrier 175, which is provided at least on one side with a coating 176. The coating 176 is, for example, a Plasmapore coating, in which high-purity titanium powder is applied to the carrier 175 by a vacuum method. This improves its osteointegrative properties in that a rough, microporous titanium layer is formed on the carrier 175, which leads to improved adhesion to the intervertebral bodies 17, 18. At least the side of the carrier 175 facing the spinous processes 15, 16 is coated.

The fixing element 174 can have at least one defined deformation region, as was described above with the aid of the fixing element 100.

The deformation region is, for example, a bending zone 177 in order to facilitate an adaptation of the fixing element to the spinous processes 15, 16 and the spacer element(s) of the respective fixation system. The bending zones 177, of which the fixing element 174 has four bending zones 177 running in parallel relative to one another in pairs, are for example formed as grooves 178 running in the carrier 175, in particular with a round cross section.

The carrier 175 is advantageously not coated in the region of the bending zones 177. This can reduce the danger of the coating 176 being detached from the carrier 175 during the deforming of the fixing element 174.

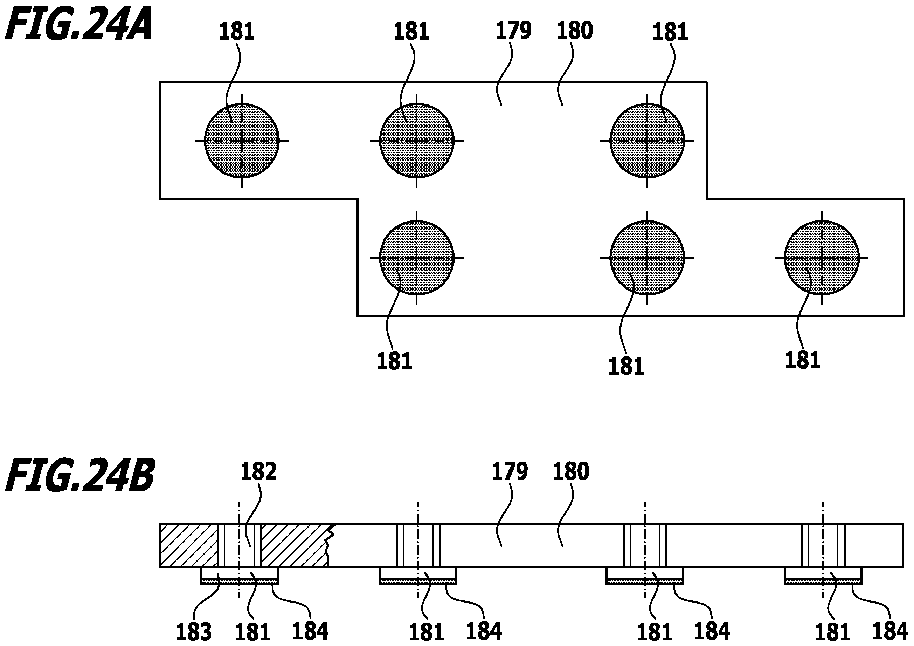

A further fixing element 179 is shown in plan view and in side view respectively in Figs. 24A and 24B. The fixing element 179 can be used in each of the fixation systems described here. It comprises a carrier 180, the base form of which is the same as that of the carrier 175, and a plurality of coating members 181. The coating members 181 are manufactured separately from the carrier 180 and fixed thereon, for example by gluing, screwing, latching, welding etc.

In the present case, each coating member 181 comprises an anchoring portion 182 for fixing in the plate of the carrier 180 and a coating portion 183 on the upper side of the plate of the carrier 180, on which the actual coating 184 is applied. The coating 184 may, for example, be a Plasmapore coating. The cross section of the coating portion 183 may, for example, be circular (Fig. 24A). It may be provided that coating members 181 with a different size, cross section of the coating portion 183 and different type and extent of the respective coating 184 can be used in the fixing element 179.

The coating 184 faces the vertebral bodies 17 and 18 and, in particular, the spinous processes 15 and 16 in order to improve the osteointegrative properties of the fixing element 179. The use of coating members 181 allows the osteointegrative properties of the fixing element 179 to be improved in a targeted manner at a defined position of said fixing element. For example, no coating member 181 is present in the region of the intervertebral space 26, in the present case.

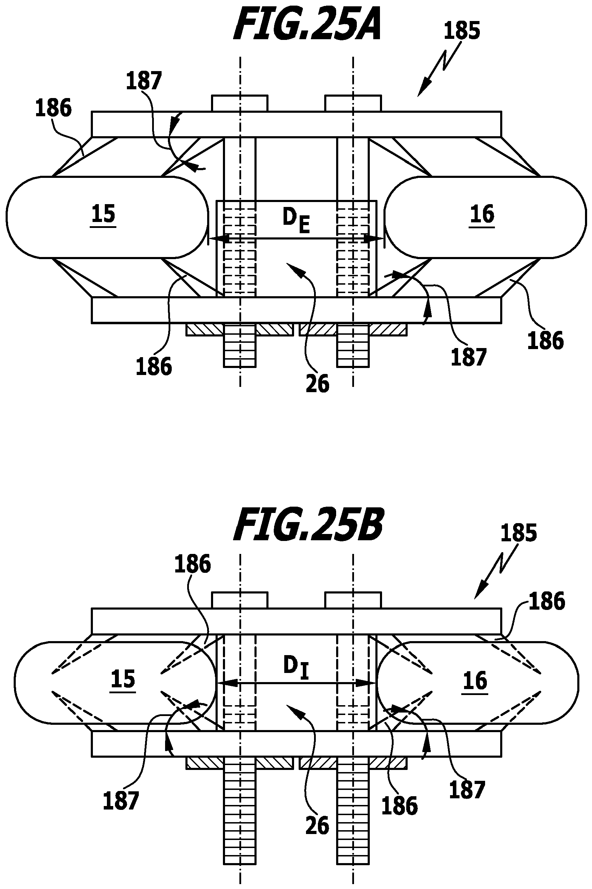

A thirteenth preferred embodiment of the fixation system according to the disclosure is shown schematically in Figs. 25A and 25B in the insertion position and the implantation position, respectively. The fixing elements, the securing device for the securing thereof in the implantation position of the fixation system 185 and the spacer element are, in this case, those of one of the fixation systems described above. The special feature of the fixation system 185 is that the fixing elements in each case have engagement members in the form of projections 186 which have an angle 187 with respect to the planes defined by the fixing elements.

The angle 187 is, for example, approximately more than 45°, measured on the side of the respective projection 186 remote from the intervertebral space 26. The use of projections 186 running obliquely with respect to the fixing elements makes it possible to exert a tensile force in the cranial-caudal direction bringing the spinous processes 15 and 16 closer to one another during the transfer of the fixation system 185 from the insertion position into the implantation position. This results in the intervertebral space 26 shortening, it being possible for the spinous processes in the insertion position of the fixation system 185 to have a spacing D_{E} from one another and a spacing D_{I} from one another in the implantation position.

It can be provided that the projections 186 enclose the same angle 187 in each case with the fixing elements. It is also conceivable, however, for different projections 186 to enclose different angles 187 with the fixing elements.

If the projections 186 are inclined relative to the fixing elements in a counter direction, i.e. proceeding from the respective fixing element in the direction of the intervertebral space 26, a spreading apart of the spinous processes 15 and 16 can also be achieved in the cranial-caudal direction.

The arrangement of the projections 13, 173 or 186 on the fixing elements can be "staggered," i.e. projections of the fixing elements lying on mutually opposing lateral sides of the spinous processes 15, 16 have a spacing from one another in the cranial-caudal and/or dorso-ventral direction. This can prevent the spinous processes 15, 16 being weakened from both sides by engagement of the projections, so that the loading of the spinous processes 15, 16 is distributed more uniformly. This allows an even more reliable fixing of the fixation system on the spinous processes 15, 16.

The above-described concept, although described with respect to the spinous processes 15, 16 of the vertebral bodies 17, 18, can also be used to fix transverse processes adjacent to one another. Likewise, combinations of a spinous process fixing and a transverse process fixing are conceivable.

## Claims

1. Surgical fixation system for stabilising spinous processes (15, 16) of adjacent vertebral bodies (17, 18) relative to one another, comprising at least one first fixing element (139) and at least one second fixing element (140), which can be transferred relative to one another from an insertion position into an implantation position, wherein the fixing elements (139, 140) in the implantation position have a smaller spacing from one another than in the insertion position and in which implantation position the fixing elements (139, 140) are engageable laterally from different sides with at least one of the spinous processes (15, 16) in each case, and comprising a securing device (141) for securing the fixing elements (139, 140) relative to one another in the implantation position and comprising at least one spacer element (142), which can be fixed by the fixing elements (139, 140) in the implantation position thereof in the intervertebral space between the spinous processes (15, 16), wherein the at least one spacer element (142) comprises or forms a cranial contact face (145) for a spinous process (15), wherein the at least one spacer element (142) comprises or forms a caudal contact face (146) for a spinous process (16), and wherein the at least one spacer element (142) comprises or forms at least one anterior contact face for at least one lamina arcus vertebrae of at least one vertebral body (17, 18), wherein the at least one spacer element (142) comprises a first portion (143) to be inserted into the intervertebral space (26) forming the cranial contact face (145) for a spinous process (15) and the caudal contact face (146) for a spinous process (16), **characterised in that** the at least one spacer element (142) further comprises a second portion (144) and a third portion, the second portion (144) and the third portion each being arranged anterior to the first portion (143), projecting laterally from the first portion (143), having the form of a strip and having an approximately sinusoidal profile in the lateral-medial transverse sectional direction, the third portion being mirror-inverted with respect to the second portion (144) and projecting laterally from the first portion (143) in the opposite direction than the second portion (144), the second portion (144) and the third portion each forming an anterior contact face.

2. Fixation system according to claim 1, **characterised in that** the at least one spacer element (142) can be fixed in positively-locking and/or force-locking manner.

3. Fixation system according to either of the preceding claims, **characterised in that** the at least one spacer element (142) can be fixed by clamping and/or **in that** the at least one spacer element (142) engages or is engageable with at least one fixing element (139, 140) in the implantation position.

4. Fixation system according to any one of the preceding claims, **characterised in that** the at least one spacer element (142) comprises or forms at least one lateral contact face (33) for the at least one fixing element (139, 140).

5. Fixation system according to any one of the preceding claims, **characterised in that** the second portion (144) and the third portion are sized so as to extend, in the cranial-caudal direction, from the processus articularis inferior of the vertebral body (17) arranged cranially and the processus articularis inferior of the vertebral body (18) arranged caudally.

6. Fixation system according to any one of the preceding claims, **characterised in that** the at least one spacer element (142) is formed in one piece.

7. Fixation system according to any one of the preceding claims, **characterised in that** the at least one spacer element (142) is resorbable.

8. Fixation system according to any one of the preceding claims, **characterised in that** the at least one spacer element (142) is made at least partially from an osteointegrative material or is osteointegratively coated.

9. Fixation system according to any one of the preceding claims, **characterised in that** the at least one spacer element (142) is made from metal, from a plastics material, from a bone material or from a bone replacement material.

10. Fixation system according to any one of the preceding claims, **characterised in that** the at least one spacer element (142) is engaged or engageable with the securing device (141).

11. Fixation system according to claim 10, **characterised in that** the at least one spacer element (142) comprises or forms at least one opening, through which the securing device (141) engages and/or at least one recess (35, 36), in which the securing device (141) engages.

12. Fixation system according to any one of the preceding claims, **characterised in that** the securing device (141) is a latching device comprising at least one latching element (22, 23), which is fixed to one fixing element (139, 140) and on which the other fixing element (139, 140) can be latched, wherein the at least one latching element engages through the at least one spacer element (142) or engages therewith.

## Patentansprüche

1. Chirurgisches Fixationssystem zur Stabilisierung von Dornfortsätzen (15, 16) benachbarter Wirbelkörper (17, 18) relativ zueinander, umfassend mindestens ein erstes Fixierelement (139) und mindestens ein zweites Fixierelement (140), die relativ zueinander von einer Einbringstellung in eine Implantationsstellung überführbar sind, wobei die Fixierelemente (139, 140) in der Implantationsstellung einen geringeren Abstand voneinander aufweisen als in der Einbringstellung und in welcher Implantationsstellung die Fixierelemente (139, 140) lateral von unterschiedlichen Seiten jeweils mit zumindest einem der Dornfortsätze (15, 16) in Eingriff bringbar sind, sowie umfassend eine Sicherungseinrichtung (141) zur Sicherung der Fixierelemente (139, 140) relativ zueinander in der Implantationsstellung sowie umfassend mindestens ein Abstandselement (142), das mittels der Fixierelemente (139, 140) in deren Implantationsstellung im Zwischenwirbelraum zwischen den Dornfortsätzen (15, 16) fixierbar ist, wobei das mindestens eine Abstandselement (142) eine craniale Anlagefläche (145) für einen Dornfortsatz (15) umfasst oder ausbildet, wobei das mindestens eine Abstandselement (142) eine caudale Anlagefläche (146) für einen Dornfortsatz (16) umfasst oder ausbildet, und wobei das mindestens eine Abstandselement (142) mindestens eine anteriore Anlagefläche für mindestens eine lamina arcus vertebrae mindestens eines Wirbelkörpers (17, 18) umfasst oder ausbildet, wobei das mindestens eine Abstandselement (142) einen ersten Abschnitt (143) zum Einsetzen, in den Zwischenwirbelraum (26) umfasst, welcher erste Abschnitt die craniale Anlagefläche (145) für einen Dornfortsatz (15) und die caudale Anlagefläche (146) für einen Dornfortsatz (16) bildet, **dadurch gekennzeichnet, dass** das mindestens eine Abstandselement (142) weiter einen zweiten Abschnitt (144) und einen dritten Abschnitt umfasst, wobei der zweite Abschnitt (144) und der dritte Abschnitt jeweils anterior des ersten Abschnitts (143) angeordnet sind, lateral vom ersten Abschnitt (143) vorspringen, streifenförmig sind und in der lateral-medialen Querschnittsrichtung ein ungefähr sinusförmiges Profil aufweisen, wobei der dritte Abschnitt spiegelbildlich bezüglich des zweiten Abschnittes (144) ausgestaltet ist und lateral vom ersten Abschnitt (143) in entgegengesetzter Richtung wie der zweite Abschnitt (144) vorspringt, wobei der zweite Abschnitt (144) und der dritte Abschnitt jeweils eine anteriore Anlagefläche bilden.

2. Fixationssystem nach Anspruch 1, **dadurch gekennzeichnet, dass** das mindestens eine Abstandselement (142) formschlüssig und/oder kraftschlüssig fixierbar ist.

3. Fixationssystem nach einem der voranstehenden Ansprüche, **dadurch gekennzeichnet, dass** das mindestens eine Abstandselement (142) klemmend fixierbar ist und/oder dass das mindestens eine Abstandselement (142) mit mindestens einem Fixierelement (139, 140) in der Implantationsstellung in Eingriff steht oder in Eingriff bringbar ist.

4. Fixationssystem nach einem der voranstehenden Ansprüche, **dadurch gekennzeichnet, dass** das mindestens eine Abstandselement (142) mindestens eine laterale Anlagefläche (33) für das mindestens eine Fixierelement (139, 140) umfasst oder ausbildet.

5. Fixationssystem nach einem der voranstehenden Ansprüche, **dadurch gekennzeichnet, dass** der zweite Abschnitt (144) und der dritte Abschnitt so bemessen sind, dass sie sich, in der cranial-caudalen Richtung, vom processus articularis inferior des cranial angeordneten Wirbelkörpers (17) und bis zum processus articularis inferior des caudal angeordneten Wirbelkörpers (18) erstrecken.

6. Fixationssystem nach einem der voranstehenden Ansprüche, **dadurch gekennzeichnet, dass** das mindestens eine Abstandselement (142) einstückig ausgebildet ist.

7. Fixationssystem nach einem der voranstehenden Ansprüche, **dadurch gekennzeichnet, dass** das mindestens eine Abstandselement (142) resorbierbar ist.

8. Fixationssystem nach einem der voranstehenden Ansprüche, **dadurch gekennzeichnet, dass** das mindestens eine Abstandselement (142) zumindest teilweise aus einem osteointegrativen Material gefertigt ist oder osteointegrativ beschichtet ist.

9. Fixationssystem nach einem der voranstehenden Ansprüche, **dadurch gekennzeichnet, dass** das mindestens eine Abstandselement (142) aus Metall, aus einem Kunststoffmaterial, aus einem Knochenmaterial oder aus einem Knochenersatzmaterial gefertigt ist.

10. Fixationssystem nach einem der voranstehenden Ansprüche, **dadurch gekennzeichnet, dass** das mindestens eine Abstandselement (142) mit der Sicherungseinrichtung (141) in Eingriff steht oder in Eingriff bringbar ist.

11. Fixationssystem nach Anspruch 10, **dadurch gekennzeichnet, dass** das mindestens eine Abstandselement (142) mindestens eine von der Sicherungseinrichtung (141) durchgriffene Durchbrechung umfasst oder ausbildet und/oder mindestens eine Ausnehmung (35, 36), in die die Sicherungseinrichtung (141) eingreift.

12. Fixationssystem nach einem der voranstehenden Ansprüche, **dadurch gekennzeichnet, dass** die Sicherungseinrichtung (141) eine Rasteinrichtung ist, umfassend mindestens ein an einem Fixierelement (139, 140) festgelegtes Rastelement (22, 23), auf das das andere Fixierelement (139, 140) aufrastbar ist, wobei das mindestens eine Rastelement das mindestens eine Abstandselement (142) durchgreift oder in dieses eingreift.

## Revendications

1. Système de fixation chirurgical pour stabiliser des apophyses épineuses (15, 16) de corps vertébraux (17, 18) adjacents l'une par rapport à l'autre, comprenant au moins un premier élément de fixation (139) et au moins un second élément de fixation (140), qui peuvent être transférés l'un par rapport à l'autre d'une position d'insertion à une position d'implantation, dans lequel les éléments de fixation (139, 140) dans la position d'implantation ont un plus petit espace les uns par rapport aux autres que dans la position d'insertion et dans laquelle position d'implantation, les éléments de fixation (139, 140) peuvent être mis en prise latéralement à partir de différents côtés avec au moins l'une des apophyses épineuses (15, 16) dans chaque cas, et comprenant un dispositif de sécurité (141) pour fixer en toute sécurité les éléments de fixation (139, 140) les uns par rapport aux autres dans la position d'implantation et comprenant au moins un élément d'espacement (142) qui peut être fixé par les éléments de fixation (139, 140) dans leur position d'implantation dans l'espace intervertébral entre les apophyses épineuses (15, 16), dans lequel le au moins un élément d'espacement (142) comprend ou forme une face de contact crânienne (145) pour une apophyse épineuse (15), dans lequel le au moins un élément d'espacement (142) comprend ou forme une face de contact caudale (146) pour une apophyse épineuse (16) et dans lequel le au moins un élément d'espacement (142) comprend ou forme au moins une face de contact antérieure pour au moins une lamina arcus vertebrae d'au moins un corps vertébral (17, 18), dans lequel le au moins un élément d'espacement (142) comprend une première partie (143) à insérer dans l'espace intervertébral (26) formant la face de contact crânienne (145) pour une apophyse épineuse (15) et la face de contact caudale (146) pour une apophyse épineuse (16), **caractérisé en ce que** le au moins un élément d'espacement (142) comprend en outre une deuxième partie (144) et une troisième partie, la deuxième partie (144) et la troisième partie étant chacune agencées de manière antérieure par rapport à la première partie (143), faisant saillie latéralement de la première partie (143), ayant la forme d'une bande et ayant un profil approximativement sinusoïdal dans la direction transversale latérale - médiale, la troisième partie étant inversée en miroir par rapport à la deuxième partie (144), et faisant saillie latéralement à partir de la première partie (143) dans la direction opposée à la deuxième partie (144), la deuxième partie (144) et la troisième partie formant chacune une face de contact antérieure.

2. Système de fixation selon la revendication 1, **caractérisé en ce que** le au moins un élément d'espacement (142) peut être fixé par verrouillage positif et/ou par verrouillage à force.

3. Système de fixation selon l'une ou l'autre des revendications précédentes, **caractérisé en ce que** le au moins un élément d'espacement (142) peut être fixé par serrage et/ou **en ce que** le au moins un élément d'espacement (142) se met en prise avec ou peut être mis en prise avec au moins un élément de fixation (139, 140) dans la position d'implantation.

4. Système de fixation selon l'une quelconque des revendications précédentes, **caractérisé en ce que** le au moins un élément d'espacement (142) comprend ou forme au moins une face de contact latérale (33) pour le au moins un élément de fixation (139, 140).

5. Système de fixation selon l'une quelconque des revendications précédentes, **caractérisé en ce que** la deuxième partie (144) et la troisième partie sont dimensionnées afin de s'étendre, dans la direction crânienne - caudale, à partir du processus articularis inférieure du corps vertébral (17) agencée de manière crânienne et au processus articularis inférieure du corps vertébral (18) agencée de manière caudale.

6. Système de fixation selon l'une quelconque des revendications précédentes, **caractérisé en ce que** le au moins un élément d'espacement (142) est formé d'un seul tenant.

7. Système de fixation selon l'une quelconque des revendications précédentes, **caractérisé en ce que** le au moins un élément d'espacement (142) est résorbable.

8. Système de fixation selon l'une quelconque des revendications précédentes, **caractérisé en ce que** le au moins un élément d'espacement (142) est réalisé au moins partiellement à partir d'un matériau d'ostéointégration ou est recouvert par voie d'ostéointégration.

9. Système de fixation selon l'une quelconque des revendications précédentes, **caractérisé en ce que** le au moins un élément d'espacement (142) est réalisé à partir de métal, à partir d'une matière plastique, à partir d'un matériau osseux ou à partir d'un matériau de remplacement osseux.

10. Système de fixation selon l'une quelconque des revendications précédentes, **caractérisé en ce que** le au moins un élément d'espacement (142) est mis en prise ou peut se mettre en prise avec le dispositif de sécurité (141).

11. Système de fixation selon la revendication 10, **caractérisé en ce que** le au moins un élément d'espacement (142) comprend ou forme au moins une ouverture, à travers laquelle le dispositif de sécurité (141) se met en prise et/ou au moins un évidement (35, 36), dans lequel le dispositif de sécurité (141) se met en prise.

12. Système de fixation selon l'une quelconque des revendications précédentes, **caractérisé en ce que** le dispositif de fixation (141) est un dispositif de verrouillage comprenant au moins un élément de verrouillage (22, 23), qui est fixé à un élément de fixation (139, 140) et sur lequel l'autre élément de fixation (139, 140) peut être verrouillé, dans lequel le au moins un élément de verrouillage se met en prise par le biais du au moins un élément d'espacement (142) ou se met en prise avec ce dernier.
